# EUROPEAN PATENT SPECIFICATION

(11) **EP 1 324 032 B1**
(45) Date of publication and mention of the grant of the patent: **07.12.2005**
(21) Application number: 01830824.7
(22) Date of filing: 28.12.2001
(51) Int. Cl.: G01N 29/22, G01N 33/46

(54) **Method and device for testing the presence of defects in the production of plane boards**
Verfahren und Vorrichtung zur Fehlerprüfung bei der Herstellung von Plattenmaterialien
Procédé et dispositif de détection de défauts dans la production de panneaux

(43) Date of publication of application: 02.07.2003
(73) Proprietor: Imal S.R.L., 41010 San Damaso, Modena (IT)
(72) Inventor: Benedetti, Paolo, 41100 Modena MO (IT)
(74) Representative: Celestino, Marco

(56) References cited:
- DE-A- 3 633 556
- DE-C- 19 519 669
- FR-A- 2 488 996
- GB-A- 2 062 866
- PATENT ABSTRACTS OF JAPAN vol. 1998, no. 01, 30 January 1998 (1998-01-30) & JP 09 229911 A (HITACHI CONSTR MACH CO LTD), 5 September 1997 (1997-09-05)

## Description

### Field of the invention

The present invention relates to a method for testing the presence of defects in the production of plane boards.

Furthermore the invention relates to an apparatus that carries out this method.

In particular, it relates to an improved appliance that can be associated to an apparatus used for testing the correct bonding of the board parts with one another, already existing in the field of production of plane boards, for example wood based boards, especially chipboards or timber fibre that can be agglomerated with concrete, plaster and the like.

### Description of the prior art

A particularly felt problem in the field of boards is the process of verification of the correct bonding of plane sheet elements of which they are made. The tests that control that the board examined has not significant defects, in fact, verify that the product fulfils quality requirements.

According to the prior art, the devices used for bonding quality control purposes, throughout or at the end of the production process, provide one or more ultrasonic pulses source and one or more receivers, arranged at opposite sides with respect to the board to analyse. If the board to analyse has a bonding defect, an ultrasonic signal is detected, that crosses it in a remarkably weakened way, by a receiver that is located opposite with respect to a sender that has generated it. Discontinuity of the signal received is an indication of the presence of defects.

However, the major problems of the known systems appear when measuring the bonding defects at the edges of the board, owing to the so called "edge effect". The edge effect is due to the capacity of the spherical sound waves to turn around the obstacles, following preferred trajectories in the directions where the resistance to propagation is minimum.

An "edge effect" tends to reduce the efficiency of the actual ultrasonic devices that test the boards for identification of defects (cracks, blisters, etc.), that, on the other hand, are often located just near the edge of the board same. Normally, with the existing ultrasonic inspection systems, qualitative data are not available on the plane board up to a distance of about 50-70 mm from the edge. In addition, the "edge effect", besides affecting an individuation of the defect near the edge, causes an undetermined amplification of the signal. In fact, as already said, the waves can follow an alternative trajectory that does not cross the board but goes around the edge same.

Furthermore, the known machines are substantially influenced by the ground noise typical of a working environment, like the fluctuation and the vibrations that are created when conveying the boards in the production process.

Finally, even far from the edges, the ultrasonic pulses systems of known art cannot determine with precision the area of presence of the defect, since the beam of ultrasonic pulses examined encounters the boards according to a rather large area. For this reason, defects of small dimensions, normally less than 50mm of diameter, cannot be detected.

A device for localised ultrasonic inspections is disclosed in document JP09229911.

### Summary of the invention

It is an object of the present invention to provide a device for testing the correct bonding of plane boards or similar components that allows to reduce the "edge effect" at the minimum.

It is another object of the present invention to provide a device for testing the correct bonding of plane boards in order to contain the vibrations and the fluctuations that could affect the analysis.

It is a particular object of the present invention to provide a device for testing the correct bonding of plane boards that allows the individuation of the actual sampling area of the board.

These and other objects are achieved by the apparatus for testing the quality requirements of wood based plane boards during their production process, according to the present invention, wherein the boards are conveyed in a control region that comprises, at opposite sides, a plurality of senders and receivers of ultrasonic waves. Each sender and receiver analyses the boards at a measured control area.

The characteristic of the apparatus according to the present invention is that a plane of reference is provided on which the boards slide, permeable to the ultrasonic waves only at a predetermined control region, thus shielding zones that surround said region.

The plane of reference, advantageously, has an hole at each control region, for defining a predetermined trajectory for the spherical ultrasonic waves in their path from each sender to the corresponding receiver. In particular, at least a part of the holes is arranged at the side edges of the board. As a consequence, it is possible to test the presence of defects (cracks, blisters, etc.) within the structure of a board, also when these defects extend up to the edges of the board same.

Furthermore the holes of the plane of reference have diameter that allows the individuation of the actual portion of board that is under inspection.

The plane of reference can be suitably mounted movable on supports, according to directions transversal to the direction of movement of the board to analyse, allowing thus its side movement integrally with the senders-receivers.

According to a particular aspect of the invention, the plane of reference is made in more parts, that are separated longitudinally and that can be moved transversally towards/away from each other, for adapting the positions of the holes of the reference plane according to the width of the board, in particular for inspecting the edges of the board. Furthermore the supports, on which the senders and the receivers are arranged, have discontinuity zones so that they can be adjusted in order to align always the senders and the receivers with the holes of the plane of reference.

Advantageously, the plane of reference has more layers, for increasing the capacity of shielding the signals that are not useful for the measure.

A further characteristic of the board is to be stiff, made preferably of wooden suitably hardened material, in order to limit the vibrations and the fluctuations that would affect the analysis.

### Brief description of the drawings

Further characteristics and the advantages of the apparatus for testing the quality of plane boards, according to the present invention, will be made clearer with the following description of an embodiment thereof, exemplifying but not limitative, with reference to the attached drawings wherein:
- figure 1 shows a longitudinal cross section of a traditional testing device, in particular showing an "edge effect" at the head portion of the board;
- figure 2 shows a transversal cross section of a traditional testing device, in particular showing an "edge effect" on an side edge of the board;
- figure 3 shows a longitudinal cross section of the apparatus according to the invention, in particular the plane of sliding is shown that avoids the "edge effect" at the head portion of figure 1;
- figure 4 shows a transversal cross section of the apparatus according to the invention, in particular how the plane of sliding avoids the "edge effect" is shown at the head portion of figure 1;
- figure 5 shows a diagrammatical view of the device that allows to move, transversally to the direction of movement of the board, the planes of support and the couples of senders-receivers.

### Description of a preferred embodiment

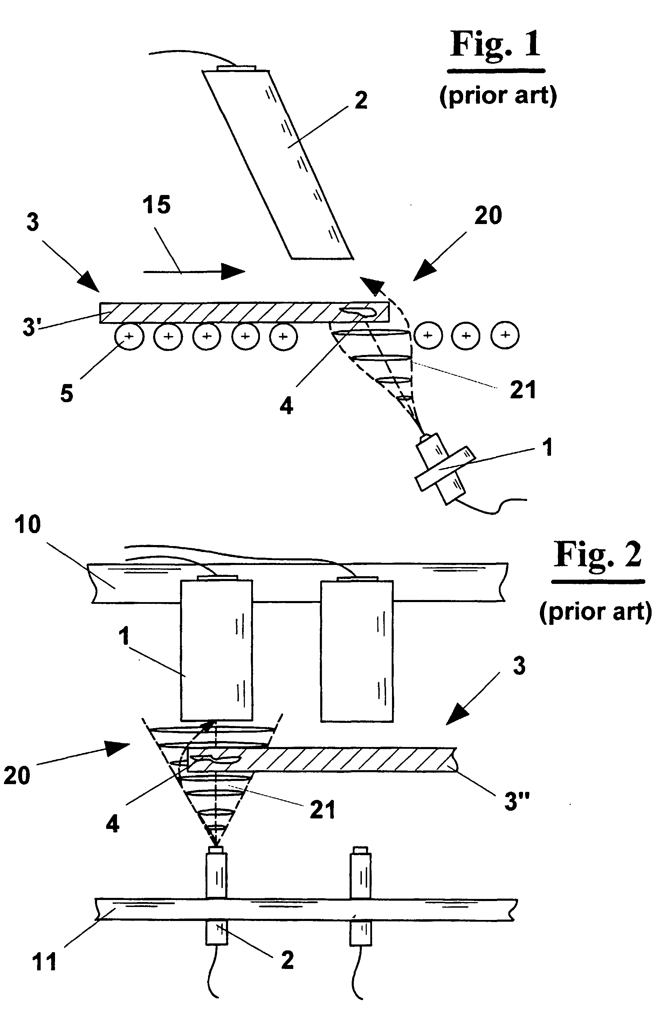

In figures 1 and 2 a traditional apparatus is shown for testing the quality of plane boards 3, for example boards completely in wooden material or wooden boards agglomerated with concrete, gesso or the like.

The apparatus is inserted in a conveying system of the plane board 3 according to a direction 15, having a row of rollers 5, and comprises a plurality of senders 1 and a plurality of corresponding receivers 2 of ultrasonic waves 21. The senders 1 and the receivers 2 are located at opposite sides with respect to the conveyor, the rollers 5 and the board 3. In figure 1, the board 3 is shown cross sectioned in longitudinal direction and presents to the waves 21 the front and rear 3' edges, whereas in figure 2 the board 3 is shown cross sectioned in a transversal direction and presents to the waves 21 the side edges 3'' (of which only one is shown).

In particular, figures 1 and 2 illustrate the major problem of the traditional systems, i.e. the so called "edge effect" 20, at the front or rear 3' edge (figure 1) and at the side edge 3" of board 3 (figure 2). The drawbacks of the "edge effect" have been described above in the introductory part.

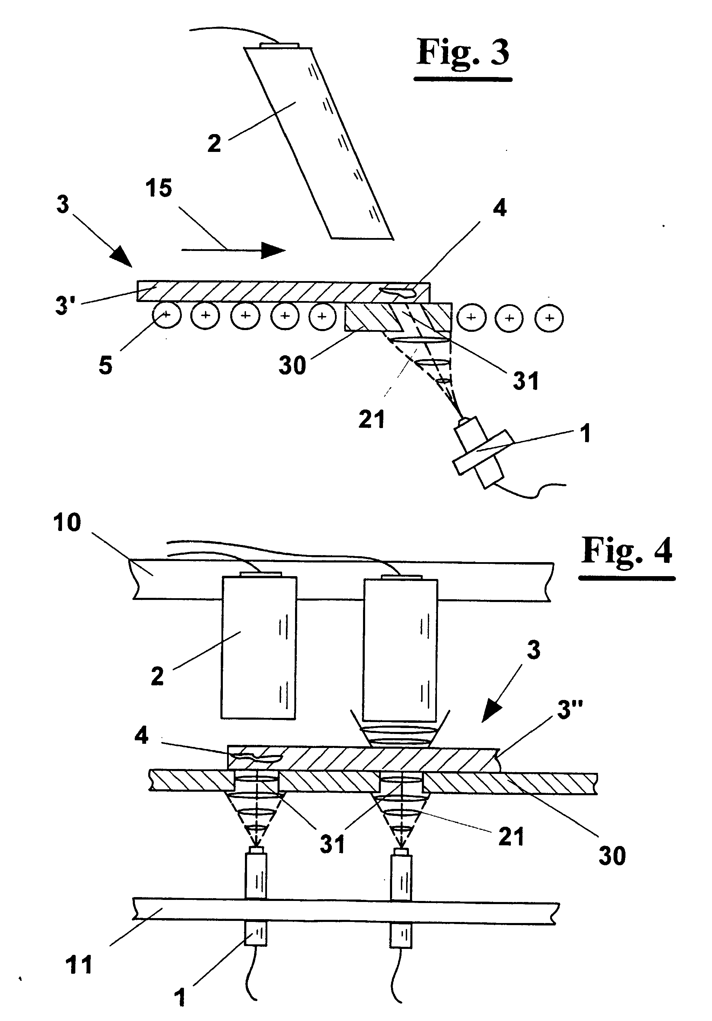

Figures 3 and 4 show the main characteristic of the apparatus according to the present invention, i.e. the presence of a plane of reference 30 on which the boards slide 3, capable of shielding the ultrasonic waves 21 at a predetermined control region.

The plane of reference 30 presents, in fact, a plurality of holes 31 that define each a respective inspection area on board 3, defining a predetermined trajectory for the spherical ultrasonic waves 21 in their path from each sender 1 to the corresponding receiver 2.

In particular, when coming across a defect, for example a recess 4, the ultrasonic waves 21 do not reach the respective receiver 2, or they reach it in a way remarkably weakened, whereas a "sound" board allows the propagation of the waves 21 up to achieving the receiver 2 with a known attenuation.

As shown in figures 3 and 4, a group of holes 31 is arranged just at the edges 3' and 3" of the board 3, thus leading the ultrasonic waves 21 to cross the edges same. As a consequence, it is possible to detect the presence of defects 4 (cracks, blisters, etc.) within the structure of a board, also when they extend up to the edges of board 3 same, with reliability up to 0-5 mm from the side edges 3", i.e. practically reducing to a small portion the side edges area that have not been inspected. Concerning the front and rear edges there is an increase of the zones that can be inspected, respectively 60% and 30%e more than the prior art. For example, with a speed of 3-4 m/s, when inspecting a board that is in movement, the zone not inspected of the boards is reduced to 30ö50mm at the rear edge and to only 20ö30mm from the front edge.

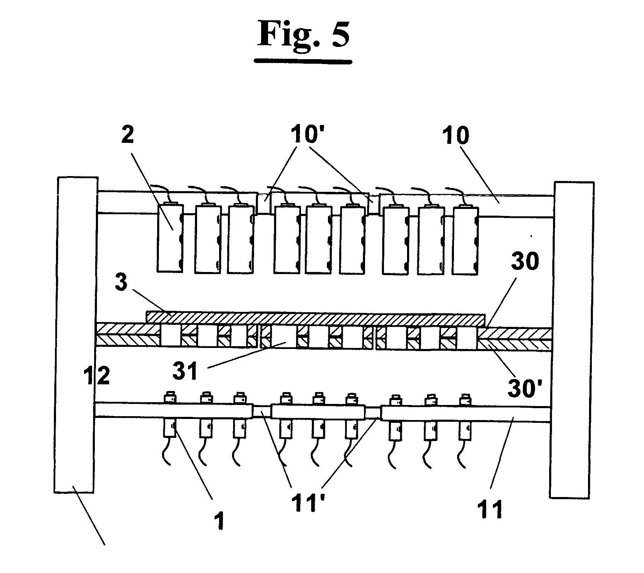

With reference to figure 5, the device for testing the quality of plane boards 3 provides that the senders 1 and the receivers 2 are mounted on supports 10 and 11, movable on a carriage 12 according to the directions that are transversal to the direction of movement 15 of board 3.

According to the invention, also the plane of reference 30, that can be coupled to a parallel plane 30', is integral to carriages 12 so that the holes 31 are always aligned to the respective senders/receivers 1 and 2.

The combination of two planes 30 and 30' is advantageous for increasing the shielding capability of the portion of signal that is not useful for the inspection. In fact, the use of two coupled boards 30, 30' that have a minimum gap of air between them allows a considerable reduction of the noise.

Furthermore, the planes of reference 30 and 30', according to an advantageous embodiment of the apparatus, when boards of different width have to be inspected, are made in two parts, that can be approached transversally or moved away from each other, for adapting the positions of the holes 31 of plane of reference 30 responsive to the width of board 3, allowing, in particular, an accurate analysis of the edges of board 3 for every different width. For this reason, the supports 10 and 11 are discontinued in 10' and 11', so that they can be adjusted in order to align the senders 1 and the receivers 2 always with holes 31. In particular, the support 10 and 11 would be made of three parts, of which the central is fixed, and the two side parts are movable on a guide, either manually or electrically by means of a servomotor.

Alternatively, it is possible also that the holes on plane of reference 30, 30' are fixed, and several frequencies of senders and receivers 1 and 2 are installed (couples TX/RX), so that they cover a board 3 of maximum width. When the couples of senders and receivers that are external to the board are turned off, when the width is narrower, it is possible to analyse a board according to a correct width.

## Claims

1. Apparatus for testing the quality of plane boards (3), in particular wooden boards, with a conveying system (5) for conveying said boards (3) into a control region that comprises at opposite sides a plurality of senders (1) and receivers (2) of ultrasonic waves (21) respectively, each sender (1) - for and receiver (2) for analysing the boards (3) in a corresponding control area comprised in said control region, **characterised in that** in said control region a plane of reference (30) is provided, on which said boards (3) can slide, suitable for shielding said ultrasonic waves (21), at said control areas said plane having a plurality of holes (31) for defining a predetermined trajectory for the spherical ultrasonic waves (21) in their path from each sender (1) to the corresponding receiver (2).

2. Apparatus for testing the quality of plane boards (3), according to the previous claims, wherein the holes (31) of said plane of reference (30) have each a limited cross section that defines a predetermined inspection area on said board (3).

3. Apparatus for testing the quality of plane boards (3), according to any one of the previous claims, wherein said plane of reference (30), senders (1) and receivers (2) of ultrasonic waves (21) are mounted on planes that are movable according to a direction transversal to the direction of movement of said board (3), wherein any transversal movement of said planes is integral to said senders (1) and receivers (2).

4. Apparatus for testing the quality of plane boards (3), according to any one of the previous claims, wherein said plane of reference (30) has at least three portions separated longitudinally, said portions being movable transversally towards/away from each other for adapting the positions of the holes (31) of said plane of reference (30) according to the width of said board (3), in particular for analysing the edges (3') of the board (3).

5. Apparatus for testing the quality of plane boards (3), according to any one of the previous claims, wherein senders (1) and receivers (2) are mounted on supports (10) and (11), which have discontinuity zones (10') and (11') so that they can be adjusted in order to align the senders (1) and the receivers (2) with said holes (31).

6. Apparatus for testing the quality of plane boards (3), according to any one of the previous claims, wherein said plane of reference (30) consists in at least two layers (30') suitable for increasing the waves shielding capability outside of said control areas.

7. Apparatus for testing the quality of plane boards (3), according to claim 1, wherein several senders (1) and receivers (2) are installed in couples according to different frequencies and cover a board (3) of maximum width, whereby an inspection on an actual width of a board (3) is possible turning off all the couples external to said actual width.

8. Apparatus for testing the quality of plane boards (3) according to any one of the preceding claims, wherein said plane of reference (30) is made of hardened timber and the like, in order to contain the vibrations and the fluctuations that could affect the test.

9. Method for testing the quality of wooden plane boards (3) during their production process, said boards (3) being conveyed (5) in a control region that comprises at opposite sides a plurality of senders (1) and receivers (2) of ultrasonic waves (21), each sender (1) and receiver (2) analysing the boards (3) in a corresponding control area in said control region, **characterised in that,** in said control region said boards (3) slide on a plane of reference (30) that is adapted to shield said ultrasonic waves (21), at said control areas said plane having holes (31) for defining a predetermined trajectory for the ultrasonic spherical waves (21) in their path from each sender (1) to the corresponding receiver (2).

10. Method for testing the quality of plane boards (3), according to claim 9, wherein at least a part of said holes (31) is arranged at the side edges (3'') of said board (3).

## Patentansprüche

1. Vorrichtung zur Prüfung der Qualität von ebenen Brettern (3), insbesondere von Holzbrettern, mit einem Fördersystem (5) zur Förderung der Bretter in einen Kontrollabschnitt, der an einander gegenüberliegenden Seiten jeweils eine Vielzahl von Sendern (1) und Empfängern (2) für Ultraschallwellen (21) aufweist, wobei jeder Sender (1) und Empfänger (2) zur Analyse der Bretter (3) in einem korrespondierenden Kontrollbereich dient, der in dem Kontrollabschnitt enthalten ist, **dadurch gekennzeichnet, dass** in dem Kontrollabschnitt eine Reverenzebene (30) vorgesehen ist, auf der die Bretter (3) gleiten können, die geeignet ist zum Abschirmen der Ultraschallwellen (21), wobei die Ebene in den Kontrollbereichen eine Vielzahl von Löchern (31) zur Definierung einer festgelegten Trajektorie für die späherischen Ultraschallwellen (21) auf deren Weg von jedem Sender (1) zu dem korrespondierenden Empfänger (2) aufweist.

2. Vorrichtung zur Prüfung der Qualität von ebenen Brettern nach dem vorstehenden Anspruch, wobei die Löcher (31) der Referenzebene (30) jeweils einen begrenzten Querschnitt haben, der einen festgelegten Inspektionsbereich auf dem Brett (3) definiert.

3. Vorrichtung zur Prüfung der Qualität von ebenen Brettern (3) nach einem der vorstehenden Ansprüche, wobei die Referenzebene (30), die Sender (1) und die Empfänger (2) für Ultraschallwellen (21) an Ebenen befestigt sind, die gemäß einer Richtung, die quer zu der Bewegungsrichtung des Brettes (3) ist, bewegbar sind, wobei jede Querbewegung der Ebenen integral mit den Sendern (1) und Empfängern (2) ist.

4. Vorrichtung zur Prüfung der Qualität von ebenen Brettern (3) nach einem der vorstehenden Ansprüche, wobei die Referenzebene (30) mindestens drei in Längsrichtung getrennte Abschnitte aufweist, wobei die Abschnitte in Querrichtung zueinander oder weg von einander zur Anpassung der Positionen der Löcher (31) der Referenzebene (30) gemäß der Breite des Brettes (3) bewegbar sind, insbesondere zur Analyse der Kanten (3') des Brettes (3).

5. Vorrichtung zur Prüfung der Qualität von ebenen Brettern (3) nach einem der vorstehenden Ansprüche, wobei die Sender (1) und Empfänger (2) an Trägern (10) und (11) befestigt sind, die Unterbrechungszonen (10') und (11') aufweisen, so dass sie eingestellt werden können, um die Sender (1) und die Empfänger (2) mit den Löchern (31) in Einklang zu bringen.

6. Vorrichtung zur Prüfung der Qualität von ebenen Brettern (3) nach einem der vorstehenden Ansprüche, wobei die Referenzebene (30) aus zumindest zwei Schichten (30') besteht, die zur Erhöhung der Wellenabschirmfähigkeit außerhalb der Steuerbereiche geeignet sind.

7. Vorrichtung zur Prüfung der Qualität von ebenen Brettern (3) nach Anspruch 1, wobei mehrere Sender (1) und Empfänger (2) paarweise gemäß verschiedener Frequenzen installiert sind und ein Brett (3) maximaler Breite abdecken, wobei eine Inspektion einer tatsächlichen Breite eines Bretts (3) möglicherweise alle Paare außerhalb der tatsächlichen Breite ausblendet.

8. Vorrichtung zur Prüfung der Qualität von ebenen Brettern (3) gemäß einem der vorstehenden Ansprüche, wobei die Referenzebene (30) aus gehärtetem Bauholz und der gleichen gefertigt ist, um die Vibrationen und die Fluktuationen zu enthalten, die die Prüfung beeinflussen können.

9. Verfahren zur Prüfung der Qualität von ebenen Holzbrettern (3) während deren Herstellungsprozess, wobei die Bretter (3) in einen Kontrollabschnitt gefördert werden (5), der an einander gegenüberliegenden Seiten eine Vielzahl von Sendern (1) und Empfängern (2) für Ultraschallwellen (21) aufweist, wobei jeder Sender (1) und Empfänger (2) die Bretter (3) in einem korrespondierenden Kontrollbereich in dem Steuerabschnitt analysiert, **dadurch gekennzeichnet, dass** in dem Kontrollabschnitt die Bretter (3) auf einer Referenzebene (30) gleiten, die ausgelegt ist, um die Ultraschallwellen (21) abzuschirmen, wobei die Ebene in den Kontrollbereichen Löcher (31) zur Definierung einer festgelegten Trajektorie für die sphärischen Ultraschallwellen (21) auf deren Weg von jedem Sender (1) zu dem korrespondierenden Empfänger (2) hat.

10. Verfahren zur Prüfung der Qualität von ebenen Brettern (3) nach Anspruch 9, wobei zumindest ein Teil der Löcher (31) an den Seitenkanten (3'') des Brettes (3) vorgesehen ist.

## Revendications

1. Appareil destiné à contrôler la qualité de panneaux plans (3), en particulier de panneaux de bois, avec un système de transport (5) destiné à transporter lesdits panneaux (3) dans une zone de contrôle qui comprend, sur des côtés opposés, une pluralité d'émetteurs (1) et de récepteurs (2) d'ultrasons (21) respectivement, chaque émetteur (1) et récepteur (2) étant destiné à analyser les panneaux (3) sur une surface de contrôle correspondante comprise dans ladite zone de contrôle, **caractérisé en ce que** dans ladite zone de contrôle un plan de référence (30) sur lequel lesdits panneaux (3) peuvent coulisser est prévu, qui est adapté pour masquer lesdits ultrasons (21), ledit plan comportant une pluralité d'orifices (31) au niveau desdites surfaces de contrôle afin de définir une trajectoire prédéterminée pour les ultrasons sphériques (21) sur leur trajet depuis chaque émetteur (1) vers le récepteur correspondant (2).

2. Appareil destiné à contrôler la qualité de panneaux plans (3) selon les revendications précédentes, dans lequel les orifices (31) dudit plan de référence (30) ont chacun une coupe transversale limitée qui définit une surface d'inspection prédéterminée sur ledit panneau (3).

3. Appareil destiné à contrôler la qualité de panneaux plans (3) selon l'une quelconque des revendications précédentes, dans lequel ledit plan de référence (30), lesdits émetteurs (1) et lesdits récepteurs (2) d'ultrasons (21) sont montés sur des plans qui peuvent se déplacer dans un sens transversal par rapport au sens de déplacement dudit panneau (3), dans lequel tout déplacement transversal desdits plans est effectué en bloc avec lesdits émetteurs (1) et lesdits récepteurs (2).

4. Appareil destiné à contrôler la qualité de panneaux plans (3) selon l'une quelconque des revendications précédentes, dans lequel ledit plan de référence (30) présente au moins trois parties séparées dans le sens de la longueur, lesdites parties pouvant se déplacer transversalement vers/à l'écart l'une de l'autre afin d'adapter les positions des orifices (31) dudit plan de référence (30) selon la largeur dudit panneau (3), en particulier pour analyser les bords (3') du panneau (3).

5. Appareil destiné à contrôler la qualité de panneaux plans (3) selon l'une quelconque des revendications précédentes, dans lequel les émetteurs (1) et les récepteurs (2) sont montés sur des supports (10) et (11) qui présentent des zones de discontinuité (10') et (11') de sorte qu'ils puissent être ajustés de manière à aligner les émetteurs (1) et les récepteurs (2) sur lesdits orifices (31).

6. Appareil destiné à contrôler la qualité de panneaux plans (3) selon l'une quelconque des revendications précédentes, dans lequel ledit plan de référence (30) consiste en au moins deux couches (30') adaptées pour accroître la capacité de masquer les ultrasons en dehors desdites surfaces de contrôle.

7. Appareil destiné à contrôler la qualité de panneaux plans (3) selon la revendication 1, dans lequel plusieurs émetteurs (1) et récepteurs (2) sont installés par paires selon des fréquences différentes et couvrent un panneau (3) d'une largeur maximale, de sorte qu'une inspection sur une largeur réelle d'un panneau (3) peut être effectuée en éteignant toutes les paires externes à ladite largeur réelle.

8. Appareil destiné à contrôler la qualité de panneaux plans (3) selon l'une quelconque des revendications précédentes, dans lequel ledit plan de référence (30) est fait de bois durci et similaire, afin de maîtriser les vibrations et les fluctuations qui pourraient affecter l'essai.

9. Procédé destiné à contrôler la qualité de panneaux de bois plans (3) au cours de leur procès de production, lesdits panneaux (3) étant transportés (5) dans une zone de contrôle qui comprend sur des côtés opposés, une pluralité d'émetteurs (1) et de récepteurs (2) d'ultrasons (21) respectivement, chaque émetteur (1) et récepteur (2) analysant les panneaux (3) dans une surface de contrôle correspondante dans ladite zone de contrôle, **caractérisé en ce que**, dans ladite zone de contrôle, lesdits panneaux (3) coulissent sur un plan de référence (30) qui est adapté pour masquer lesdits ultrasons (21), ledit plan comportant des orifices (31) au niveau desdites surfaces de contrôle afin de définir une trajectoire prédéterminée pour les ultrasons sphériques (21) sur leur trajet depuis chaque émetteur (1) vers le récepteur correspondant (2).

10. Procédé destiné à contrôler la qualité de panneaux plans (3) selon la revendication 9, dans lequel au moins une partie desdits orifices (31) est disposée au niveau des bords latéraux (3 ") dudit panneau (3).
